(19) Europäisches Patentamt
European Patent Office
Office européen des brevets

(11) **EP 4 509 820 A1**

(12) **EUROPEAN PATENT APPLICATION**
published in accordance with Art. 153(4) EPC

(43) Date of publication:
**19.02.2025 Bulletin 2025/08**

(21) Application number: **23803613.1**

(22) Date of filing: **11.05.2023**

(51) International Patent Classification (IPC):
***G01N 21/64*** (2006.01)      ***G01N 21/21*** (2006.01)
***G01N 33/543*** (2006.01)

(52) Cooperative Patent Classification (CPC):
**G01N 21/21; G01N 21/64; G01N 33/543**

(86) International application number:
**PCT/JP2023/017784**

(87) International publication number:
**WO 2023/219139 (16.11.2023 Gazette 2023/46)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority:  **13.05.2022  JP 2022079524**
**08.05.2023  JP 2023076794**

(71) Applicants:
• **CANON KABUSHIKI KAISHA**
**Tokyo 146-8501 (JP)**
• **Canon Medical Systems Corporation**
**Tochigi 324-0036 (JP)**

(72) Inventors:
• **KAKEGAWA Norishige**
**Tokyo 146-8501 (JP)**

• **MASUMURA Takahiro**
**Tokyo 146-8501 (JP)**
• **YAMAUCHI Fumio**
**Tokyo 146-8501 (JP)**
• **KANAZAKI Kengo**
**Tokyo 146-8501 (JP)**
• **NAKAMURA Tomohiro**
**Tokyo 146-8501 (JP)**
• **NAKAJIMA Ikuo**
**Tokyo 146-8501 (JP)**
• **SAKAKIBARA Teigo**
**Tokyo 146-8501 (JP)**

(74) Representative: **WESER & Kollegen**
**Patentanwälte PartmbB**
**Radeckestraße 43**
**81245 München (DE)**

(54) **ANALYSIS METHOD AND ANALYSIS DEVICE EMPLOYING MEASUREMENT BASED ON POLARIZATION ANISOTROPY**

(57)    Provided are an analysis method and an analysis apparatus each of which enables the analysis of the concentration of a target substance in a wide concentration range and with high sensitivity based on polarization anisotropy. Specifically, provided is an analysis method including measuring a value (R) for polarization anisotropy through use of a luminescent reagent that binds to a target substance, to thereby calculate a concentration of the target substance, the analysis method including: a mixing step of mixing a sample containing the target substance and the luminescent reagent to provide a mixed liquid; and a measuring step of measuring the R of the mixed liquid after a lapse of a time T from a time of the mixing, wherein in the measuring step, a time at which the R reaches R1 is represented by T1, and the concentration of the target substance is calculated based on the T1.

EP 4 509 820 A1

# FIG. 1

MIXING STEP OF MIXING SAMPLE CONTAINING
TARGET SUBSTANCE AND LUMINESCENT
REAGENT TO PROVIDE MIXED LIQUID

MEASURING STEP OF MEASURING VALUE (R)
FOR POLARIZATION ANISOTROPY
(REPEATEDLY PERFORMED AT PERIOD OF (a+b) SECONDS)

$R \geqq R1$

NO

YES

T1

**Description**

[Technical Field]

The present invention relates to an analysis method and an analysis apparatus based on polarization anisotropy.

[Background Art]

In the fields of medicine and clinical tests, high-sensitivity detection or quantification of a trace amount of a biological component from, for example, blood or a collected part of an organ is required for investigating, for example, the cause and presence or absence of a disease.

Among test techniques for biological components, immunoassays are widely utilized. In many of the immunoassays, a washing step called bound/free (B/F) separation is required. As an immunoassay that does not require the B/F separation, there is known a latex agglutination method utilizing an antigen-antibody reaction. In the latex agglutination method, latex particles each having supported thereon, for example, an antibody that specifically binds to a target substance are mixed with a liquid that may contain the target substance, and the degree of agglutination of the latex particles is measured.

In the latex agglutination method, the target substance is captured by the antibody bound to the latex particles and specific to the target substance, and a plurality of the latex particles are crosslinked via the captured target substance, with the result that the agglutination of the latex particles occurs. That is, the amount of the target substance in a liquid sample such as a biological sample can be quantified by evaluating the degree of the agglutination of the latex particles. The degree of the agglutination can be quantified by measuring and evaluating a change in amount of light transmitted through or scattered by the liquid sample.

The latex agglutination method can detect and quantitatively evaluate an antigen serving as the target substance in a simple and rapid manner, but has involved a problem with detection limits in that the antigen cannot be detected when its amount in the liquid sample such as the biological sample is small.

To improve the detection sensitivity for the target substance, it is required that the degree of the agglutination be measured with higher sensitivity. That is, it is conceivable to replace a system for measuring the change in amount of the light transmitted through or scattered by the liquid sample with a detection/quantification method utilizing a luminescence characteristic with higher sensitivity. Specifically, there has been proposed, for example, a specimen test method utilizing a fluorescence depolarization method (Patent Literatures 1 and 2).

In Patent Literature 1, it is proposed that an apparatus for the fluorescence depolarization method be improved to be clinically used.

In the fluorescence depolarization method, the B/F separation required in a general fluorescence measurement method is not required.

Accordingly, the use of the fluorescence depolarization method enables a simple specimen test as with the latex agglutination method. Further, it is conceived that the use of the fluorescence depolarization method enables measurement by the same test system as that of the latex agglutination method by merely mixing a luminescent substance that specifically reacts with the target substance in a measurement process. Meanwhile, in Patent Literature 1, there is a proposal of the use of a single molecule such as fluorescein as a luminescent material, which is applicable only to a drug, a low-molecular-weight antigen, and the like in principle.

Patent Literature 2 has solved the problem of Patent Literature 1, i.e., the problem in that the fluorescence depolarization method is applied only to a drug, a low-molecular-weight antigen, and the like. That is, in Patent Literature 2, with an aim to apply the fluorescence depolarization method to a macromolecule such as a protein, it is proposed to use, as a luminescent material, a material obtained by causing latex particles to adsorb a dye having a long-lifetime luminescence characteristic. In Patent Literature 2, it is proposed that a high-molecular-weight substance be quantified by balancing a reduction in rotational Brownian motion of the substance in a liquid due to an increase in particle diameter and the length of emission lifetime based on the principle of the fluorescence depolarization method. However, in Patent Literature 2, a fluorescent substance is supported on the latex particles after synthesis of the particles, and hence an interaction between fluorescent substances adsorbed in the vicinity of surfaces of the particles or the like makes it difficult to stably determine the polarization anisotropic property of the testing particles. Further, in Patent Literature 2, bovine serum albumin (BSA), which is a biomolecule, is supported on surfaces of the particles in order to suppress nonspecific adsorption, and hence a lot-to-lot variation may occur owing to a broad particle size distribution and BSA, which is a protein.

Accordingly, measurement is performed at the concentration of the target substance in the order of μg/mL, which is not greatly different from the latex method in terms of measurement sensitivity.

Further, in Patent Literature 2, there are proposals of a method of measuring a difference between polarization anisotropic properties in agglutination between particles before and after a specific reaction time, and a method of evaluating the degree of the agglutination based on the value of a polarization anisotropic property at the end point of the lapse of the reaction time.

In the measurement method of Patent Literature 2, the upper limit value of the polarization anisotropic property is determined by a luminescent substance to be used as a probe. Accordingly, when the measurement is performed in a wide range, the amount of a reagent needs to be adjusted in accordance with the concentration of the target substance. In other words, a concentration range that is measurable with one kind of reagent has been limited.

Accordingly, even in Patent Literature 2, it has been hard to say that the detection of the target substance with high sensitivity and in a wide concentration range can be achieved.

[Citation List]

[Patent Literature]

PTL 1: Japanese Patent Publication No. H03-52575
PTL 2: Japanese Patent No. 2893772

[Summary of Invention]

[Technical Problem]

The present invention has been made in view of such related art, and an object of the present invention is to provide an analysis method and an analysis apparatus each of which enables the analysis of the concentration of a target substance in a wide concentration range and with high sensitivity based on polarization anisotropy.

[Solution to Problem]

According to one embodiment of the present invention, there is provided an analysis method including measuring a value (R) for polarization anisotropy through use of a luminescent reagent that binds to a target substance, to thereby calculate a concentration of the target substance,
the analysis method including:

a mixing step of mixing a sample containing the target substance and the luminescent reagent to provide a mixed liquid; and
a measuring step of measuring the R of the mixed liquid after a lapse of a time T from a time of the mixing,
wherein in the measuring step, a time at which the R reaches R1 is represented by T1, and the concentration of the target substance is calculated based on the T1, and
wherein when the R measured for the luminescent reagent that is free from being mixed with the target substance is represented by R0, R1>R0 is satisfied.

In addition, according to one embodiment of the present invention, there is provided an analysis apparatus configured to measure a value (R) for polarization anisotropy through use of a luminescent reagent that binds to a target substance, to thereby calculate a concentration of the target substance,
the analysis apparatus including:

a mixing portion configured to mix a sample containing the target substance and the luminescent reagent to provide a mixed liquid;
a measuring portion configured to measure the R of the mixed liquid after a lapse of a time T from a time of the mixing; and
a controlling portion,
wherein when a time at which the R reaches R1 in the measuring portion is represented by T1, the controlling portion is configured to calculate the concentration of the target substance based on the T1,
provided that when the R measured for the luminescent reagent that is free from being mixed with the target substance is represented by R0, R1>R0 is satisfied.

[Advantageous Effects of Invention]

According to the analysis method of the embodiment of the present invention, a change in polarization anisotropy can be detected with high sensitivity in correspondence to the aggregation/dispersion behavior of particles, and hence the amount of the target substance can be measured in a wide concentration range. According to the analysis apparatus of the embodiment of the present invention, the target substance can be detected and quantified with high sensitivity and over a wide concentration range based on the polarization anisotropy.

Further features of the present invention will become apparent from the following description of exemplary embodiments with reference to the attached drawings.

[Brief Description of Drawings]

[FIG. 1]

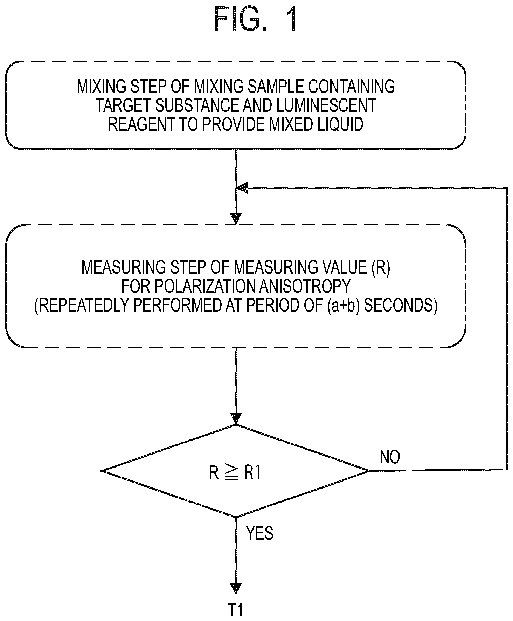

FIG. 1 is a schematic view for illustrating an analysis method according to an embodiment of the present invention.
[FIG. 2]

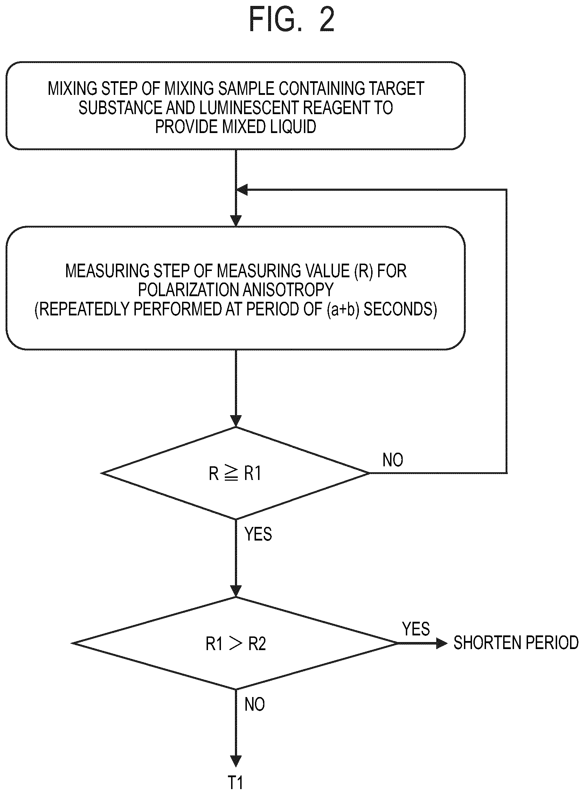

FIG. 2 is a schematic view for illustrating the analysis method according to the embodiment of the present invention.
[FIG. 3]

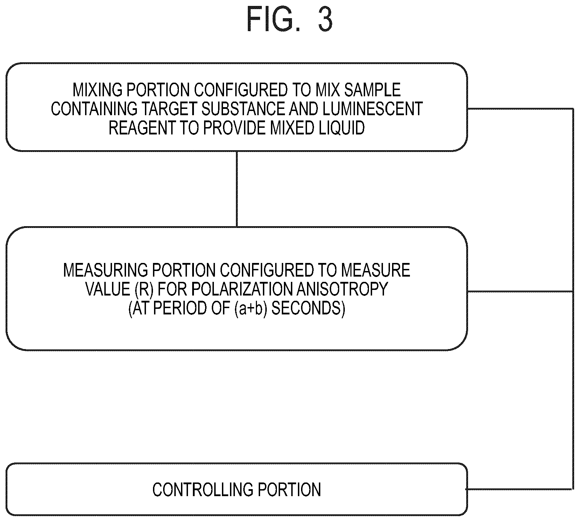

FIG. 3 is a schematic view for illustrating an apparatus according to an embodiment of the present invention.
[FIG. 4]

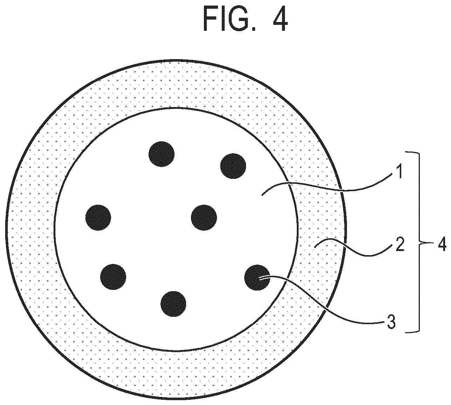

FIG. 4 is a schematic view for illustrating a luminescent reagent to be used in the embodiment of the present invention.
[FIG. 5]

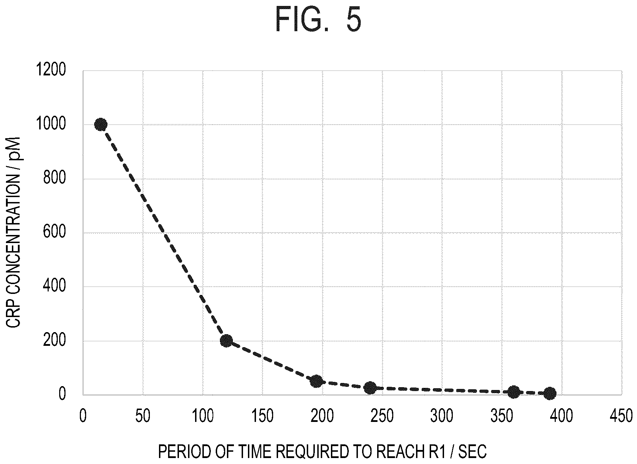

FIG. 5 is a graph showing evaluation results, that is, showing the results of the quantification of a CRP antigen concentration by the method according to the embodiment of the present invention.

[Description of Embodiments]

Preferred embodiments of the present invention are described in detail below. However, the embodiments are not intended to limit the scope of the present invention.

According to one embodiment of the present invention, there is provided the following analysis method: an analysis method including measuring a value (R) for polarization anisotropy through use of a luminescent reagent that binds to a target substance, to thereby calculate a concentration of the target substance, the analysis method including: a mixing step of mixing a sample containing the target substance and the luminescent reagent to provide a mixed liquid; and a measuring step of measuring the R of the mixed liquid after a lapse of a time T from a time of the mixing, wherein in the measuring step, a time at which the R reaches R1 is represented by T1, and the concentration of the target substance is calculated based on the T1, and wherein when the R measured for the luminescent reagent that is free from being mixed with the target substance is represented by R0, R1>R0 is satisfied.

(Value for Polarization Anisotropy)

In this embodiment, the value for polarization anisotropy (sometimes referred to as R) is defined as described below. That is, the R is a value indicating a relationship between the luminescence intensity of a polarized light component in a parallel direction to radiated polarized light and the luminescence intensity of a polarized light component in a perpendicular direction thereto regarding luminescence generated by exciting a luminescent substance through irradiation with polarized light. More specifically, the R is a value calculated from the luminescence intensity of a luminescence component having a vibration direction parallel to that of given polarized light and the luminescence intensity of a luminescence component having a vibration direction orthogonal (perpendicular) to that of the polarized light, the luminescence intensities being determined when the luminescent substance is excited by the polarized light. Further, the R is a value indicating the ratio of a difference between the luminescence intensity of a luminescence component having a vibration direction parallel to that of a first polarized light beam at the time of excitation by the first polarized light beam and the luminescence intensity of a luminescence component having a vibration direction orthogonal to that of the first polarized light beam at the time of excitation by the first polarized light beam to the sum of the luminescence intensities. The R may be corrected with: a ratio between the luminescence intensity of a luminescence component having a vibration direction orthogonal to that of a second polarized light beam having a vibration direction orthogonal to that of the first polarized light beam at the time of excitation by the second polarized light beam and the luminescence intensity of a luminescence component having a vibration direction parallel to that of the second polarized light beam having a vibration direction orthogonal to that of the first polarized light beam at the time of excitation by the second polarized light beam; and other constants. The value for polarization anisotropy encompasses values referred to as "polarization anisotropic property," "degree of polarization," and the like.

More specifically, for example, the R may be "r" in the following equation (1).

[Math. 1]

$$r = \frac{I_{VV} - G * I_{VH}}{I_{VV} + 2 * G * I_{VH}}$$
$$G = \frac{I_{HV}}{I_{HH}} \qquad \cdots (1)$$

(in the equation (1),

$I_{VV}$ represents the luminescence intensity of a luminescence component having a vibration direction parallel to that of a first polarized light beam at the time of excitation by the first polarized light beam,

$I_{VH}$ represents the luminescence intensity of a luminescence component having a vibration direction orthogonal to that of the first polarized light beam at the time of excitation by the first polarized light beam,

$I_{HV}$ represents the luminescence intensity of a luminescence component having a vibration direction orthogonal to that of a second polarized light beam having a vibration direction orthogonal to that of the first polarized light beam at the time of excitation by the second polarized light beam,

$I_{HH}$ represents the luminescence intensity of a luminescence component having a vibration direction parallel to that of the second polarized light beam having a vibration direction orthogonal to that of the first polarized light beam at the time of excitation by the second polarized light beam, and

G represents a correction value.)

In addition, the R may be r' in the following equation (2).
[Math. 2]

$$r' = \frac{I_{VV} - G * I_{VH}}{I_{VV} + G * I_{VH}}$$
$$G = \frac{I_{HV}}{I_{HH}} \qquad \cdots (2)$$

Each symbol is the same as that in the equation (1).

With regard to conditions for the measurement of the R, for example, it is preferred that the measurement be performed in a liquid having a temperature of 0°C or more and 50°C or less, and that the viscosity of the liquid be 0.5 mPa·s or more and 50 mPa·s or less. When the luminescent reagent contains europium complex-containing particles, the measurement is preferably performed at a concentration of the luminescent reagent of 0.001 mg/ml or more and 0.1 mg/ml or less, and an excitation wavelength is preferably 500 nm or more and 700 nm or less.

(With Regard to R1 and R0)

The R1 may be appropriately defined by an analyst in accordance with the purpose of the measurement and the properties of the target substance.

For example, when the analysis of the concentration of the target substance with high sensitivity (in a low-concentration range) is targeted, R1-R0≥0.0001 is preferably satisfied. For example, to analyze the target substance in the measurement sample in the measurement range of from 1 pM or more to 10 nM or less, R1-R0≥0.0001 is preferably satisfied. The maximum value of the R1-R0 is 0.4, which is a theoretical value determined from the configuration of the analysis method.

Meanwhile, when the analysis of the concentration of the target substance with low sensitivity (in a high-concentration range) is targeted, R1-R0 is preferably set low. For example, to analyze the target substance in the measurement sample in the measurement range of from 1 nM or more to 1 M or less, R1-R0<0.0001 may be satisfied.

The R1 may be determined as follows: a measurer measures the value for polarization anisotropy through use of various standard samples having known concentrations in advance to produce a calibration curve; and the R1 is determined from the curve.

In addition, with regard to the R0, which is the R measured for the luminescent reagent that is not mixed with the target substance, R0≥0.001 is preferably satisfied.

(With Regard to Mixing Step)

Reference may be made to FIG. 1 for the respective steps. In the mixing step, the sample containing the target substance and the luminescent reagent are mixed to provide the mixed liquid. The mixed liquid is a liquid containing the luminescent reagent and the target substance, and may further contain, for example, an additive except the foregoing. The mixing is preferably performed in the pH range of from 3.0 or more to 11.0 or less. In addition, a mixing temperature falls within the range of from 20°C or more to 50°C or less. The target substance and the luminescent reagent are described later.

(With Regard to Measuring Step)

In the measuring step, the R of the mixed liquid after the lapse of the time T from the time of the mixing is measured. The term "time of the mixing" as used herein is not limited to the time at which the sample and the luminescent reagent are brought into contact with each other, and may be a time after the lapse of a predetermined period of time from the time at which the sample and the reagent are brought into contact with each other to the extent that the measurement is properly performed. For example, the time of the mixing may be any one of: a time after the lapse of a predetermined period of time (e.g., 10 seconds) from the time at which the sample and the luminescent reagent are brought into contact with each other; the time at which a stirring step, which is performed after the sample and the luminescent reagent have been brought into contact with each other, is completed; and a time after the lapse of a predetermined period of time from the time at which the stirring step is completed.

With regard to conditions for the measurement, for example, it is preferred that the measurement be performed in a liquid having a temperature of 0°C or more and 50°C or less, and that the viscosity of the liquid be 0.5 mPa·s or more and 50 mPa·s or less. The measurement is preferably performed at a concentration of the luminescent reagent of 0.0001 mg/ml or more and 0.1 mg/ml or less, and an excitation wavelength is preferably 500 nm or more and 700 nm or less.

When a certain amount of the target substance is present in the target substance, the R of the mixed liquid increases with a reaction time.

The increase rate of the numerical value of the R depends on the concentration of the target substance in the target substance. Specifically, when the concentration of the target substance is high, the increase rate of the R value is large, and when the concentration is low, the increase rate reduces.

That is, when the concentration of the target substance is high, the reaction time at which the value of the polarization anisotropic property R reaches the R1 is short, and when the concentration of the target substance is low, the reaction time lengthens.

The time at which the R becomes just the R1 is not necessarily a time during the measurement. Accordingly, the T1 can be determined through estimation from the time at which a value close to the R1 is measured.

The T1 can be determined through calculation from measurement results. For example, when the mixed liquid is subjected to the measurement at certain time intervals, the increase rate of the polarization anisotropic property R can be calculated with respect to the reaction time. That is, the time at which the R value of the mixed liquid that is being measured reaches the R1 can be determined through calculation from the gradient of a change in R with respect to the reaction time.

When the value of the R1 can be predicted, a measurement time can be shortened. Accordingly, the analysis method may be suitably used when the concentration of the target substance is low.

In addition, the value of the polarization anisotropic property R1 may be determined later. A quantitative evaluation can be performed by using the following step: after data obtained by plotting a change in R with respect to the T has been measured, the numerical value of the R1 is determined, and is compared to a similarly measured result of the standard substance.

The use of the analysis method of this embodiment enables the determination of the concentration of the target substance in a wide range without preparation of many test reagents in accordance with the concentration of the target substance. In general, the preparation of many reagents for the same kind of target substance in an analysis apparatus often becomes a problem because the preparation is not only complicated but also affects a setting space or the like. In addition, when the concentration exceeds a measurement concentration range, and hence the quantification of the substance ends in failure, the dilution and preparation of a specimen need to be performed again, and hence cost for one measurement increases. Accordingly, the fact that a small number of kinds of reagents can cover a wide measurement range has an important value in the performance of a specimen test.

It is preferred that the measuring step be repeatedly performed. As illustrated in FIG. 1, when the R does not reach the R1, the measuring step may be further performed.

The measuring step to be repeatedly performed may be periodically repeated, and may be repeated at a period of, for example, (a+b) seconds where "a" represents a period of time required for the measurement and "b" represents a measurement interval. That is, one measurement takes "a" seconds, and then, after an interval of "b" seconds has been placed, the next measurement may be started. For example, the "a" may be set to 1 millisecond or more and 10 seconds or less, and the "b" may be set to 1 second or more and 1 minute or less.

For example, the a+b may be set to preferably 1 second or more and 10 minutes or less, more preferably 1 second or more and 1 minute or less, still more preferably 15 seconds or more and 30 seconds or less.

In the measuring step to be repeatedly performed, when the R exceeds the R1 for the first time in an n-th measuring step, for example, any numerical value between the ending time of an (n-1)-th measuring step and the starting time of an (n+1)-th measuring step may be adopted as the T1.

(With Regard to R2)

The upper limit value (R2) of the R may be defined in advance. That is, when the R exceeds even the R2 in the period in which the R exceeds the R1 for the first time, the period is preferably adjusted by shortening at least one of the "a" or the "b" (FIG. 2). For example, a case in which the concentration of the target substance in the mixed liquid is high, and hence the value of the polarization anisotropic property R reaches the R2 in a slight period of time from the start of a reaction corresponds to the foregoing. In such case, the quantification of the high-concentration liquid is performed as follows: the "a" or the "b" is shortened, and the R is newly measured, followed by the quantification based on a calibration curve matching the R.

The R2 may be set to, for example, the R to be measured when the target substance is added to such an extent that the luminescent reagent is saturated.

Similarly, when the concentration of the target substance in the mixed liquid is low, and hence an increase in R value is slow, the quantification of the low-concentration liquid may be performed by performing the following step: the "a" or the "b" is lengthened, and the value for polarization anisotropy is measured, followed by comparison between a calibration curve matching the value and a measurement result.

When conditions for the step of quantifying the target substance through comparison to the calibration curve are classified in accordance with the reaction time as described above, a wide measurement range can be covered with a small number of kinds of reagents.

**In** addition, according to a further embodiment of the present invention, there is provided the following measurement apparatus: a measurement apparatus configured to measure a value (R) for polarization anisotropy through use of a luminescent reagent that binds to a target substance, to thereby measure a concentration of the target substance, the measurement apparatus including: a mixing portion configured to mix a sample containing the target substance and the luminescent reagent to provide a mixed liquid; a measuring portion configured to measure the R of the mixed liquid after a lapse of a time T from a time of the mixing; and a controlling portion, wherein when a time at which the R reaches R1 in the measuring portion is represented by T1, the controlling portion is configured to calculate the concentration of the target substance based on the T1, provided that when the R measured for the luminescent reagent that is free from being mixed with the target substance is represented by R0, R1>R0 is satisfied.

(Target Substance)

Examples of the target substance may include an antigen, an antibody, a low-molecular-weight compound, various receptors, an enzyme, a substrate, a nucleic acid, a cytokine, a hormone, a neurotransmitter, a transmitter, and a membrane protein. Examples of the antigen include an allergen, a bacterium, a virus, a cell, a cell membrane constituent, a cancer marker, various disease markers, an antibody, a blood-derived substance, a food-derived substance, a natural product-derived substance, and any low-molecular-weight compound. Examples of the nucleic acid include DNA, RNA, or cDNA derived from a bacterium, a virus, or a cell, a part or fragment thereof, a synthetic nucleic acid, a primer, and a probe. Examples of the low-molecular-weight compound include a cytokine, a hormone, a neurotransmitter, a transmitter, and a membrane protein, and receptors therefor. The analysis method according to this embodiment may determine the presence or absence of the target substance by comparing the concentration of the target substance to a predetermined threshold. For example, the target substance may be determined to be present when the concentration of the target substance is equal to or higher than the predetermined threshold, and to be absent when the concentration is lower than the predetermined threshold.

(Luminescent Reagent)

In this embodiment, the luminescent reagent is a reagent that causes luminescence, in particular, a reagent that emits light by being excited through photoirradiation, and a reagent based on luminescence caused by a chemical reaction such as luminol is excluded. The luminescence, which includes phosphorescence and fluorescence, is preferably phosphorescence. In this embodiment, the luminescent reagent more preferably contains a europium complex. In addition, in this embodiment, the luminescent reagent more preferably contains particles. In this embodiment, the luminescent reagent most preferably contains europium complex-containing particles. In addition, the luminescent reagent preferably has a ligand specific to the target substance. The presence of the ligand enables the particles of this embodiment to detect and quantify the target substance based on polarization anisotropy. In this embodiment, the ligand refers to a compound that specifically binds to a specific target substance.

Any compound that shows affinity for a particular substance may be used as the ligand. Examples of the ligand and the target substance, or a combination of the target substance and the ligand may include the following. That is, the examples may include: an antigen and an antibody; a low-molecular-weight compound and a receptor therefor; an enzyme and a substrate; and nucleic acids complementary to each other. Further, the examples may include an antibody and any of the following substances specific thereto: an allergen, a bacterium, a virus, a cell, a cell membrane constituent, a cancer marker, various disease markers, an antibody, a blood-derived substance, a food-derived substance, a natural product-derived substance, and any low-molecular-weight compound. Further, the examples may include a receptor and any of the following substances specific thereto: a low-molecular-weight compound, a cytokine, a hormone, a neurotransmitter, a transmitter, and a membrane protein. Further, the examples may include DNA, RNA, or cDNA derived from a bacterium, a virus, or a cell, a part or fragment thereof, a synthetic nucleic acid, a primer, or a probe, and a nucleic acid having complementarity thereto. Other than the foregoing, any combination known to have affinity may be used as the combination of the target substance and the ligand. A typical example of the ligand in this embodiment is any one of an antibody, an antigen, and a nucleic acid.

FIG. 4 is a schematic view for illustrating an example of the luminescent reagent to be used in this embodiment. A luminescent reagent 4 to be used in this embodiment preferably includes: a particle substrate 1 containing a europium complex 3; and a hydrophilic layer 2 coating the surface thereof. The diameter of the luminescent reagent in FIG. 4 is 25 nm or more and 500 nm or less.

The diameter of each of the particles may be determined by a dynamic light scattering method. When particles dispersed in a solution are irradiated with laser light and the resultant scattered light is observed with a photon detector, an intensity distribution due to the interference of the scattered light is constantly fluctuating because the particles are constantly shifting their positions by Brownian motion.

The dynamic light scattering method is a measurement method for observing the state of the Brownian motion as a fluctuation in scattered light intensity. The fluctuation of scattered light with respect to time is expressed as an autocorrelation function, and a translational diffusion coefficient is determined. A Stokes diameter is determined from the determined diffusion coefficient, and the size of each of the particles dispersed in the solution can be derived.

The luminescent reagent desirably has nothing provided on the surfaces of its particles from the viewpoint of keeping the uniformity and monodispersity of the particles. However, for the purpose of use in the analysis method according to this embodiment, nonspecific adsorption of substances except the target onto the particles needs to be prevented, and hence the luminescent reagent preferably includes a hydrophilic layer for keeping its surface hydrophilic.

A method including supporting BSA on the surface of each of the particles is widely used as a technique for keeping hydrophilicity, but this method may cause a lot variation. In view of the foregoing, the luminescent reagent preferably includes a hydrophilic layer containing a hydrophilic polymer. The concentration of the luminescent reagent in the mixed liquid is preferably 0.000001 mass% or more and 1 mass% or less, more preferably 0.00001 mass% or more and 0.001 mass% or less.

The luminescent reagent to be used in this embodiment contains the europium complex, and hence can emit phosphorescence having a long lifetime. In the luminescent reagent to be used in this embodiment, an average particle diameter that is the average of the diameters of the particles is preferably 25 nm or more and 500 nm or less, and the average particle diameter is more preferably 50 nm or more and 300 nm or less. When the average particle diameter is more than 500 nm, the polarization anisotropic property before aggregation becomes high, resulting in a small difference from the polarization anisotropic property after the aggregation reaction. In addition, when the average particle diameter is less than 25 nm, a change between sizes before and after the aggregation becomes small to make it difficult to grasp the change of the R through phosphorescent luminescence depolarization.

By reducing the particle size distribution of the luminescent reagent and introducing the europium complex as the luminescent molecule, a change in polarized luminescence characteristic can be grasped even when the dispersion state of the particles in the liquid undergoes a slight change. Specifically, even in the case where the concentration of the target substance in the solution is from about a nanogram to about a picogram, for example, 1 picogram or more and 100 picograms or less, per mL, when the molecules of the luminescent reagent aggregate via the target substance, a change in rotational Brownian motion of the luminescent reagent can be grasped as a change in polarization anisotropy.

The "polarized luminescence" refers to the following phenomenon: when a luminescent material having an anisotropic property in transition moment (transition dipole moment) uses polarized light along its transition moment as excitation light, its luminescence is also polarized light along the transition moment. The europium complex shows fluorescent luminescence based on energy transfer from the ligand to the central metal ion, and hence the transition moment is complicated, but red luminescence around 610 nm, which is derived from electronic transition from the lowest excited state $5D0$ to $7F2$, is emitted as polarized light.

The principle of polarization anisotropy is the measurement of a shift in transition moment based on the rotational motion of a luminescent material during the occurrence of polarized luminescence. The rotational motion of the luminescent material may be expressed by the equation (3):

$$Q = 3V\eta/kT \cdots (3)$$

where Q represents the rotational relaxation time of the material, V represents the volume of the material, $\eta$ represents the viscosity of a solvent, k represents the Boltzmann constant, and T represents an absolute temperature.

The rotational relaxation time of the material is a period of time required for a molecule to rotate by an angle $\theta$ (68.5°) at which $\cos\theta = 1/e$.

It is found from the equation (3) that the rotational relaxation time of the luminescent material is proportional to the volume of the material, that is, when the luminescent material has a particulate shape, the cube of the particle diameter. Meanwhile, a relationship between the emission lifetime of the luminescent material and the degree of polarization serving as the value for polarization anisotropy may be expressed by the equation (4):

$$p0/p = 1 + A(\tau/Q) \cdots (4)$$

where p0 represents a degree of polarization at a time when the material is stationary (Q=∞), "p" represents the degree of polarization, A is a constant, $\tau$ represents the emission lifetime of the material, and Q represents the rotational relaxation time.

It is found from the equation (3) and the equation (4) that the degree of polarization is influenced by the emission lifetime of the luminescent material and the rotational relaxation time, that is, the volume (particle diameter) of the luminescent material, i.e., influenced by the balance between the particle diameter and emission lifetime of the luminescent material.

When the degree of polarization of the luminescent material represented by the equation (4) is determined experimentally, it is appropriate that polarized light be caused to enter the luminescent material, and luminescence be detected in a 90° direction with respect to the traveling direction and vibration direction of excitation light. **In** this case, it is appropriate that the detected light be detected by being divided into polarized light components in parallel and perpendicular directions with respect to the polarized light that is the incident light, and for example, a polarization anisotropic property represented by the equation (5) be adopted as the value for polarization anisotropy:

$$R(t) = (I\|(t) - GI\perp(t))/(I\|(t) + 2GI\perp(t)) \cdots (5)$$

where R(t) represents a polarization anisotropic property at a time "t", $I\|(t)$ represents the luminescence intensity of a luminescence component parallel to the excitation light at the time "t", $I_\perp(t)$ represents the luminescence intensity of a luminescence component perpendicular to the excitation light at the time "t", and G represents a correction value, the ratio of $I_\perp/I\|$ measured with excitation light having a vibration direction different by 90° from that of the excitation light used for sample measurement.

That is, when the particle size and the emission lifetime fall within appropriate ranges, a change in size of the luminescent material through, for example, a reaction with the target substance can be sensitively read as a change in polarization anisotropic property. That is, the R(t) of the unaggregated luminescent material is observed to be low, and the R(t) of the aggregated luminescent material is observed to be high. This is the principle of polarization anisotropy.

The value for polarization anisotropy may be corrected with G and 2G, or may be a value without G and 2G.

(Particle Substrate 1)

FIG. 4 is an illustration of an example of the luminescent reagent 4 having a spherical shape, and the luminescent reagent 4 includes the particle substrate 1. In FIG. 4, an example in which the luminescent reagent 4 and the particle substrate 1 both have spherical shapes is illustrated, but the shapes of the luminescent reagent 4 and the particle substrate 1 to be used in this embodiment are not limited. The particle substrate 1 is not particularly specified as long as the particle substrate 1 is a material capable of stably incorporating the europium complex, but is preferably a polymer containing a styrene unit and an organic silane unit. **In** particular, for example, a polymer obtained by polymerizing a composition containing styrene as a main component and a radically polymerizable organic silane is suitably used. When the composition contains styrene as the main component, particles having an extremely uniform particle size distribution can be produced by an emulsion polymerization method to be described later. **In** addition, when a polymer containing an organic silane unit is adopted, a silanol group (Si-OH) is produced in the polymer in an aqueous solvent, and particle substrate surfaces form a siloxane bond (Si-O-Si) to each other, via which the hydrophilic layer to be described later or a ligand can be provided. The particles according to this embodiment each preferably have a ligand-bonding functional group capable of bonding a ligand to the outside of the particle substrate.

(Hydrophilic Layer 2)

The hydrophilic layer 2 may be formed by incorporating a hydrophilic polymer or a hydrophilic molecule on the outside of the particle substrate 1. The hydrophilic polymer or the hydrophilic molecule is a polymer or molecule containing a hydrophilic group, and specific examples of the hydrophilic group include molecules or polymers each having a hydroxy group, an ether, pyrrolidone, or a betaine structure. Specific examples of the hydrophilic polymer include polyethylene glycol, polyvinylpyrrolidone, a polymer of sulfobetaine, a polymer of phosphobetaine, and polyglycidyl methacrylate whose molecule has an end modified with a hydroxy group by ring-opening a glycidyl group, and those hydrophilic polymers may each be used as a main component of the hydrophilic layer 2. Alternatively, the hydrophilic layer 2 may be formed by directly providing a single molecule having a hydrophilic group on the surface of the particle substrate 1 through use of a silane coupling agent or the like. The thickness of the hydrophilic layer 2 is not limited, but does not need to be set to be large beyond a thickness with which hydrophilicity can be exhibited. When the hydrophilic layer 2 is excessively thick, the hydrophilic layer may become hydrogel-like and be hydrated by the influence of ions in the solvent, to thereby make its thickness unstable. The thickness of the hydrophilic layer 2 is suitably 1 nm or more and 15 nm or less.

(Europium Complex 3)

The luminescent reagent to be used in this embodiment may contain the europium complex 3 as a luminescent dye. The europium complex 3 has a feature in that the wavelength and intensity of its luminescence are hardly influenced by the surroundings, and hence the luminescence has a long lifetime. The europium complex 3 is made up of a europium element and a ligand. In consideration of the emission lifetime, a visible emission wavelength region, and the like, a luminescent dye is preferably a europium complex. Europium generally has an emission lifetime of 0.1 ms or more and 1.0 ms or less. The emission lifetime and the rotational relaxation time obtained from the equation (1) need to be appropriately adjusted. In the case of europium in a water dispersion, when the diameter of the luminescent reagent is 50 nm or more and 300 nm or less, the R significantly changes before and after aggregation.

At least one of the constituent ligands of the europium complex 3 is a ligand having a light-collecting function. The "light-collecting function" refers to an action of being excited at a particular wavelength to excite the central metal of the complex through energy transfer. In addition, it is preferred that the constituent ligands of the europium complex 3 include a ligand such as a $\beta$-diketone to prevent the coordination of a water molecule. The ligand such as the $\beta$-diketone coordinated to a europium ion suppresses a deactivation process due to the transfer of energy to a solvent molecule or the like to provide strong luminescence.

The europium complex 3 may be a polynuclear complex.

In addition, specific examples of the europium complex include [tris(2-thenoyltrifluoroacetone)(bis(triphenylphosphineoxide))europium(III)], [tris(2-thenoyltrifluoroacetone)(triphenylphosphineoxide)(dibenzylsulfoxide)europium(II I)], and [tris(2-thenoyltrifluoroacetone)phenanthroline)europium(III)].

At the time of a state in which the Brownian rotational motion of the europium complex 3 can be regarded as stationary in a medium, the polarization anisotropic property expressed by the equation (3) is desirably 0.08 or more. The state in which the Brownian rotational motion can be regarded as stationary refers to a state in which the rotational relaxation time of the particles is sufficiently longer than the emission lifetime of the europium complex 3.

The europium complex 3 is preferably incorporated in a larger amount into the particle substrate 1 because a luminescence intensity per particle becomes stronger. Meanwhile, when the europium complexes 3 aggregate in the particle substrate 1, an interaction between ligands influences the excitation efficiency of the europium complex 3 and the like to make it difficult to measure the polarization anisotropic property while keeping reproducibility. Whether the europium complex 3 shows non-aggregated luminescence behavior in the particle substrate 1 may be judged from an excitation spectrum of the sample.

Particles having strong luminescence not only enable high-sensitivity measurement, but also enable an increase in biochemical reaction rate because luminescence is kept even when their particle diameters are reduced. As the particle diameters become smaller, the diffusion coefficient of Brownian motion in the liquid becomes larger, and hence the reaction can be detected in a shorter period of time.

When a liquid having dispersed therein such particles is used in the analysis method according to this embodiment, a change in anisotropic property of polarized luminescence can be detected with high sensitivity in correspondence to the aggregation/dispersion behavior of the particles. A dispersion obtained by dispersing such particles in a water solvent may be utilized as a high-sensitivity test reagent using polarization anisotropy. A buffer solution may be used in the water solvent. In addition, a surfactant, an antiseptic, a sensitizer, or the like may be added into the water solvent for improving the stability of the liquid having dispersed therein the particles.

(Method of producing Luminescent Reagent)

Next, an example of a method of producing the luminescent reagent to be used in this embodiment is described.

The method of producing the luminescent reagent includes a step (first step) of mixing radically polymerizable monomers including at least styrene and a radically polymerizable organic silane, a radical polymerization initiator, a polarized luminescent europium complex, and a hydrophilic polymer with an aqueous medium to prepare an emulsion.

The method of producing the luminescent reagent includes a step (second step) of heating the emulsion to polymerize the radically polymerizable monomers.

Further, the method of producing the luminescent reagent may include a step (third step) of providing a ligand-bonding functional group to be described later on the surface of the luminescent reagent. Herein, the ligand-bonding functional group refers to a functional group that can bond a ligand. Specifically, any one of a carboxy group, an amino group, a thiol group, an epoxy group, a maleimide group, a succinimidyl group, or an alkoxysilyl group (silicon alkoxide structure) may be used.

(Radically Polymerizable Monomers)

The production of the luminescent reagent is performed by polymerizing radically polymerizable monomers, and the radically polymerizable monomers include at least styrene and a radically polymerizable organic silane. The radically polymerizable monomers may further include a monomer selected from the group consisting of: an acrylate-based monomer; and a methacrylate-based monomer. Examples of the monomer may include butadiene, vinyl acetate, vinyl chloride, acrylonitrile, methyl methacrylate, methacrylonitrile, methyl acrylate, and mixtures thereof. That is, one kind or a plurality of kinds of those monomers may be used in addition to styrene and the radically polymerizable organic silane. In addition, a monomer having two or more double bonds per molecule such as divinylbenzene may be used as a crosslinking agent.

The inclusion of the radically polymerizable organic silane in the radically polymerizable monomers provides a siloxane bond to the particle substrate 1. Examples of the radically polymerizable organic silane may include vinyltrimethoxysilane, vinyltriethoxysilane, p-styryltrimethoxysilane, 3-methacryloxypropylmethyldimethoxysilane, 3-methacryloxypropyltrimethoxysilane, 3-methacryloxypropylmethyldiethoxysilane, 3-methacryloxypropyltriethoxysilane, 3-acryloxypropyltrimethoxysilane, and combinations thereof. The use of the radically polymerizable organic silane serves to form a backbone of an inorganic oxide in the particle substrate 1 to improve the physical and chemical stability of the luminescent reagent. Further, the use of the radically polymerizable organic silane enhances affinity between the particle substrate 1 and each of the hydrophilic layer 2 and the ligand-bonding functional group.

Further, the inclusion of the radically polymerizable organic silane in the radically polymerizable monomers provides a silanol group to the surface of the particle substrate 1. The silanol group and the hydrophilic polymer such as PVP form a hydrogen bond. Thus, the hydrophilic polymer such as PVP is more strongly adsorbed onto the surface of the particle substrate 1.

(Radical Polymerization Initiator)

A wide range of compounds selected from, for example, azo compounds and organic peroxides may each be used as the radical polymerization initiator. Specific examples thereof may include 2,2'-azobis(isobutyronitrile), 2,2'-azobis(2,4-dimethylvaleronitrile), 2,2'-azobis(2-methylbutyronitrile), 4,4'-azobis(4-cyanovaleric acid), 2,2'-azobis(2-methylpropionamidine) dihydrochloride, dimethyl 2,2'-azobis(2-methylpropionate), tert-butyl hydroperoxide, benzoyl peroxide, ammonium persulfate (APS), sodium persulfate (NPS), and potassium persulfate (KPS).

(Hydrophilic Polymer)

The luminescent reagent may include a hydrophilic polymer as the hydrophilic layer. The hydrophilic polymer preferably suppresses nonspecific adsorption. Examples of the hydrophilic polymer include hydrophilic polymers each containing a unit having an ether, a betaine, or a pyrrolidone ring. It is preferred that the hydrophilic layer be included in the synthesized luminescent reagent, and be mainly present on the particle surface on the outside of the particle substrate. Herein, a polymer having a pyrrolidone ring is sometimes abbreviated as "PVP". When the PVP is fed at the time of the synthesis of the luminescent reagent, a nonspecific adsorption-suppressing ability and a ligand-bonding ability can be simultaneously provided to the luminescent reagent. The PVP to be fed at the time of the synthesis has higher hydrophilicity than those of the radically polymerizable monomers, and hence is present at an interface between the solvent and the particle substrate that is being polymerized at the time of the synthesis. The particle substrate adsorbs the PVP on the outside thereof by involving part of the PVP at the time of the polymerization, or by physical/chemical adsorption such as an interaction between a pyrrolidone ring and styrene (radically polymerizable monomer).

The molecular weight of the PVP is preferably 10,000 or more and 100,000 or less, more suitably 40,000 or more and 70,000 or less. When the molecular weight is less than 10,000, the hydrophilicity of the surface of the luminescent reagent is weak, and hence nonspecific adsorption is liable to occur. When the molecular weight is more than 100,000, the hydrophilic layer becomes so thick as to gel, thereby becoming difficult to handle.

In addition to the PVP, another hydrophilic polymer may be added as a protective colloid at the time of the synthesis of the particle substrate.

In addition, the luminescent reagent preferably satisfies A2-A1≤0.1.

A1 and A2 are defined as described below. That is, with regard to a mixture obtained by adding 30 $\mu$L of a 0.1 wt% dispersion of the luminescent reagent to 60 $\mu$L of a buffer solution mixed with 16 $\mu$L of human serum diluted 15-fold, the absorbance of the mixture immediately after the addition is represented by A1, and the absorbance of the mixture after having been left to stand at 37°C for 5 minutes after the addition is represented by A2. The absorbances are measured at an optical path of 10 mm and a wavelength of 572 nm.

Particles showing an A2-A1 of 0.1 or less have little nonspecific adsorption of impurities in serum, and are hence preferred.

(Aqueous Medium)

The aqueous medium (aqueous solution) to be used in the above-mentioned method of producing the luminescent reagent preferably contains water at 80 wt% or more and 100 wt% or less in the medium. The aqueous solvent is preferably water or a water-soluble organic solvent, and examples thereof include solutions each obtained by mixing water with methanol, ethanol, isopropyl alcohol, or acetone. When an organic solvent other than water is incorporated at more than 20 wt%, the dissolution of the polymerizable monomers may occur at the time of the production of the particles.

In addition, the aqueous medium preferably has its pH adjusted to 6 or more and 9 or less in advance. When the pH has a value of less than 6 or more than 9, the alkoxide group or silanol group of the radically polymerizable organic silane may undergo condensation polymerization or a reaction with another functional group before the formation of the polymer, leading to aggregation of the particles to be obtained. In this embodiment, the alkoxide is not intentionally subjected, before the polymerization, to condensation polymerization.

The above-mentioned pH is preferably adjusted with a pH buffer, but may be adjusted with an acid or a base.

Other than the foregoing, a surfactant, an antifoaming agent, a salt, a thickener, and the like may be used by being added at a ratio of 10% or less with respect to the aqueous medium.

In the production of the luminescent reagent, it is preferred that, first, the PVP be dissolved in the aqueous medium whose pH has been adjusted to from 6 to 9. The content of the PVP is preferably 0.01 wt% or more and 10 wt% or less, more preferably 0.03 wt% or more and 5 wt% or less with respect to the aqueous medium. When the content is less than 0.01 wt%, the amount of adsorption onto the particle substrate is small, and the effect thereof is not expressed. In addition, when the content is more than 10 wt%, the viscosity of the aqueous medium may be increased to preclude sufficient stirring.

Subsequently, the radically polymerizable monomers including the styrene (A) and the radically polymerizable organic silane (B) are added into the above-mentioned aqueous medium to prepare an emulsion. A weight ratio between the styrene (A) and the radically polymerizable organic silane (B) is from 6:4 to 100:1. Further, the prepared emulsion is mixed with the europium complex. At this time, when the solubility of the europium complex is low, a water-insoluble organic solvent may be added. A weight ratio between the europium complex and the radically polymerizable monomers is from 1:1,000 to 1:10.

When the weight ratio between the styrene (A) and the radically polymerizable organic silane (B) is less than 6:4, the specific gravity of the particles as a whole may be increased, resulting in remarkable sedimentation of the particles. In addition, to increase adhesiveness between the PVP and luminescent particles, it is desired that the weight ratio between the styrene (A) and the radically polymerizable organic silane (B) be set to 100:1 or more.

A weight ratio between the weight of the aqueous medium and the total amount of the radically polymerizable monomers is preferably from 5:5 to 9.5:0.5. When the weight ratio between the weight of the aqueous medium and the total amount of the radically polymerizable monomers is less than 5:5, remarkable aggregation of the particles to be produced may occur. In addition, when the weight ratio between the weight of the aqueous medium and the total amount of the radically polymerizable monomers is more than 9.5:0.5, the production amount of the particles may be reduced, though there is no problem with the production thereof.

The radical polymerization initiator is used by being dissolved in water, a buffer, or the like. The radical polymerization initiator may be used between 0.5 mass% or more and 10 mass% or less in the emulsion with respect to the total weight of the styrene (A) and the radically polymerizable organic silane (B).

In the above-mentioned step of heating the emulsion, it is only required that the entire emulsion be uniformly heated. A heating temperature may be arbitrarily set between 50°C or more and 80°C or less, and a heating time may be arbitrarily set between 2 hours or more and 24 hours or less. Through the heating of the emulsion, the radically polymerizable monomers are polymerized.

The luminescent reagent may have a ligand-bonding functional group on its surface. The ligand-bonding functional group is not particularly limited as long as the functional group can bond an antibody, an antigen, an enzyme, or the like, but for example, may be a carboxy group, an amino group, a thiol group, an epoxy group, a maleimide group, a succinimidyl group, a silicon alkoxide group, or the like, or contain any of those functional groups. For example, a silane coupling agent having the ligand-bonding functional group and the synthesized particles may be mixed to provide the functional group to the particle surface. Specifically, an aqueous solution of a silane coupling agent having a carboxy group may be prepared and mixed with a dispersion of the synthesized particles to provide the carboxy group to the particle surface. At this time, a dispersant such as Tween 20 may be added to the reaction solution. A reaction temperature may be arbitrarily set between 0°C or more and 80°C or less, and a reaction time may be arbitrarily set between 1 hour or more and 24 hours or less. To suppress an abrupt condensation reaction of the silane coupling agent, it is suitable that the temperature be set to be equal to or lower than a room temperature of about 25°C, and the reaction time be set to 3 hours or more and 14 hours or less. Depending on the ligand-bonding functional group, the reaction with the particle surface may be promoted by adding an acid or alkali catalyst.

The luminescent reagent can be utilized as particles for a specimen test by bonding a ligand such as any of various antibodies thereto. An optimal technique for bonding an antibody of interest or the like through utilization of a functional group present on the hydrophilic layer 2 only needs to be selected.

(Introduction of Ligand)

A hitherto known method may be applied to a chemical reaction for chemically bonding the ligand-bonding functional group and the ligand to the extent that the object of the present invention can be achieved. In addition, when the ligand is amide-bonded, a catalyst such as 1-[3-(dimethylaminopropyl)-3-ethylcarbodiimide] may be appropriately used.

The luminescent reagent to be used in this embodiment may be preferably applied to a latex immunoagglutination measurement method utilized widely in the fields of clinical tests, biochemical research, and the like.

(Analysis Apparatus)

According to one embodiment of the present invention, there is provided the following analysis method: an analysis apparatus configured to measure a value (R) for polarization anisotropy through use of a luminescent reagent that binds to a target substance, to thereby calculate a concentration of the target substance, the analysis apparatus including: a mixing portion configured to mix a sample containing the target substance and the luminescent reagent to provide a mixed liquid; a measuring portion configured to measure the R of the mixed liquid after a lapse of a time T from a time of the mixing; and a controlling portion, wherein when a time at which the R reaches R1 in the measuring portion is represented by T1, the controlling portion is configured to calculate the concentration of the target substance based on the T1, provided that when the R measured for the luminescent reagent that is free from being mixed with the target substance is represented by R0, R1>R0 is satisfied.

The outline of the analysis apparatus according to this embodiment is illustrated in FIG. 3.

The mixing portion is a portion that performs the mixing step. The measuring portion is a portion that performs the measuring step. The controlling portion controls the mixing portion and the measuring portion.

The mixing portion and the measuring portion may each include, for example, a nozzle that can suck and discharge a liquid, a container that stores various samples, a well plate, and a stage that moves these constituents. Further, the measuring portion may include an optical system including, for example, a light source, a polarizing filter, or a spectrophotometer. The controlling portion has a computer function. For example, the controlling portion may be formed integrally with a desktop personal computer (PC), a laptop PC, a tablet PC, a smartphone, or the like. To achieve a function as a computer that performs an operation and storage, the controlling portion includes a CPU, a RAM, a ROM, and a HDD. In addition, the controlling portion may include a communication interface (I/F), a display device, and an input device.

(Reagent)

The analysis method according to this embodiment may be used in a specimen test or *in vitro* diagnosis. A reagent for use in such test or diagnosis may include: the luminescent reagent to be used in this embodiment; and a dispersion medium in which the luminescent reagent is dispersed. The amount of the luminescent reagent to be incorporated into the reagent is preferably 0.000001 mass% or more and 20 mass% or less, more preferably 0.0001 mass% or more and 1 mass% or less. The reagent may include, a third substance, such as an additive or a blocking agent, in addition to the luminescent reagent to the extent that the object of the present invention can be achieved. The reagent may include a combination of two or more kinds of third substances, such as an additive and a blocking agent. Examples of the dispersion medium to be used in this embodiment include various buffer solutions, such as a phosphate buffer solution, a glycine buffer solution, a Good's buffer solution, a Tris buffer solution, and an ammonia buffer solution, but the dispersion medium included the test reagent in this embodiment is not limited thereto. When the reagent is used for the detection of an antigen or an antibody in a specimen, an antibody or an antigen may be used as the ligand.

[Examples]

The present invention is specifically described below by way of Examples. However, the present invention is not limited to these Examples.

(1) Production of Luminescent Particles

Polyvinylpyrrolidone (PVP-K30: manufactured by Tokyo Chemical Industry Co., Ltd.) was dissolved in a 2-morpholinoethanesulfonic acid (MES) buffer solution (manufactured by Kishida Chemical Co., Ltd.) having a pH of 7 to prepare a solvent A.

[Tris(2-thenoyltrifluoroacetone)(bis(triphenylphosphineoxide))europium(III)] (manufactured by Central Techno Corporation, hereinafter abbreviated as "Eu(TTA)$_3$(TPPO)$_2$") serving as a europium complex, a styrene monomer (manufactured by Kishida Chemical Co., Ltd.), 3-methacryloxypropyltrimethoxysilane (manufactured by Tokyo Chemical Industry Co., Ltd., hereinafter abbreviated as "MPS") were mixed to prepare a reaction liquid B. The reaction liquid B was added into a four-necked flask containing the solvent A, and the mixture was stirred with a mechanical stirrer set to 300 rpm. After 15 minutes of stirring under a nitrogen flow condition, the temperature of an oil bath that had been prepared was set to 70°C, and the nitrogen flow was performed for an additional 15 minutes. After the mixed liquid had been heated and stirred, an aqueous solution having dissolved therein potassium persulfate (hereinafter abbreviated as "KPS") (manufactured by Sigma-Aldrich) was added into the reaction solution, and emulsion polymerization was performed for 20 hours. After the polymerization reaction, the resultant suspension was subjected to ultrafiltration with about 4 L of ion-exchanged water through use of an ultrafiltration membrane having a molecular weight cutoff of 100K to wash the product, to thereby provide a dispersion of luminescent particles.

An aliquot of the dispersion of the luminescent particles obtained by the emulsion polymerization was taken and added to an aqueous solution having dissolved therein 1 mass% of Tween 20 (manufactured by Kishida Chemical Co., Ltd.). After 10 minutes of stirring, a silane coupling agent, X12-1135 (manufactured by Shin-Etsu Chemical Co., Ltd.), was added, and the mixture was stirred overnight. After the stirring, the dispersion was centrifuged, the supernatant was removed, and the precipitate was redispersed with pure water. The operations of centrifugation and redispersion were performed 3 or more times to wash the product. The precipitate after the washing was redispersed in pure water. Thus, ligand-bonding functional groups were introduced into particles 1 to 8. A mass ratio among the particles, pure water, and X12-1135 loaded was set to 1:300:2.

(Production of Anti-CRP Antibody-modified Luminescent Reagent)

An aliquot of 0.25 mL of the particle dispersion at 1.2 wt% corresponding to synthesized luminescent particles was taken, and the solvent was replaced by 1.6 mL of a MES buffer solution having a pH of 6.0. To the particle MES buffer solution, 1-[3-(dimethylamino)propyl]-3-ethylcarbodiimide and N-hydroxysulfosuccinimide sodium were added at 0.5 wt%, and the mixture was subjected to a reaction at 25°C for 1 hour. After the reaction, the dispersion was washed with a MES buffer solution having a pH of 5.0, an anti-CRP antibody was added at 100 μg/mL, and the anti-CRP antibody was bonded to the particles at 25°C for 2 hours. After the bonding, the particles were washed with a Tris buffer solution having a pH of 8. After the reaction, the particles were washed with a phosphate buffer solution to provide an anti-CRP antibody-modified luminescent reagent having a concentration of 0.3 wt% (sometimes referred to as "affinity particles").

The bonding of the antibodies to the particles was recognized by measuring the amount of a reduction in antibody concentration in the buffer solution having added thereto the antibodies by BCA assay.

(Preparation of Luminescent Reagent Liquid)

The resultant luminescent reagent was diluted with a phosphate (PBS) buffer solution having a pH of 7.4 so as to have a concentration of 0.1 mg/mL to prepare a luminescent reagent liquid.

(Preparation of Diluted Liquid)

A 4-(2-hydroxyethyl)-1-piperazineethanesulfonic acid (HEPES) buffer solution and a PBS buffer solution were mixed at a ratio of 1:1 in terms of volume ratio to prepare a diluted liquid.

Reaction between Target Substance and Ligand (Antigen-Antibody Reaction)

A liquid obtained by mixing a CRP antigen into a HEPES buffer solution was prepared, and was warmed to a temperature of 37°C. The luminescent reagent liquid was added to the prepared mixed liquid, and the whole was quickly stirred, followed by the observation of the polarization anisotropy of the mixed liquid. The polarization anisotropy was investigated while the concentration of the luminescent reagent was fixed at 0.01 mg/mL or 0.0025 mg/mL, and the concentration of the CRP antigen was set to from 0 pM to 1,000 pM. The observation was performed at a temperature of 37°C. The polarization anisotropy is described later.

(Example 1)

After the value of a specific polarization anisotropic property R1 had been set to 0.062, the value of a polarization anisotropic property R was measured by performing an antigen-antibody reaction with an apparatus 1 to be described later. The time at which the polarization anisotropic property R1 reached 0.062 was measured, and a time-CRP antigen concentration plot was produced, followed by the evaluation of the CRP antigen concentration. The concentration of the luminescent reagent was set to 0.01 mg/mL.

(Example 2)

A CRP antigen concentration was evaluated by the same method as that of Example 1 except that: the value of the specific polarization anisotropic property R1 was set to 0.043; and an apparatus 2 to be described later was used.

(Example 3)

The measurement of an antigen-antibody reaction was performed by the same method as that of Example 1. After that, the result of a subtraction between the numerical value of the polarization anisotropic property R 5 minutes after the start of the reaction and the R value 1 minute after the start of the reaction, and the result of a subtraction between the numerical value of the polarization anisotropic property R 40 minutes after the start of the reaction and the R value 1 minute after the start of the reaction were evaluated together.

(Example 4)

After the value of the specific polarization anisotropic property R1 had been set to 0.076, the value of the polarization anisotropic property R was measured by performing an antigen-antibody reaction with the apparatus 1 to be described later. The time at which the polarization anisotropic property R1 reached 0.076 was measured, and a time-CRP antigen concentration plot was produced, followed by the evaluation of the CRP antigen concentration. Sodium alginate was added to the diluted liquid to thicken the liquid, to thereby set the concentration of the luminescent reagent to 0.0025 mg/mL.

(Comparative Example 1)

The measurement of an antigen-antibody reaction was performed by the same method as that of Example 3, and only the result of a subtraction between the numerical value of the polarization anisotropic property R 5 minutes after the start of the reaction and the R value 1 minute after the start of the reaction was evaluated.

(Comparative Example 2)

The measurement of an antigen-antibody reaction was performed by the same method as that of Example 3, and only the result of a subtraction between the numerical value of the polarization anisotropic property R 40 minutes after the start of the reaction and the R value 1 minute after the start of the reaction was evaluated.

(Evaluations of Products)

The products in Examples and Comparative Examples were subjected to such evaluations as described below.
The shape of the product was evaluated with an electron microscope (S5500 manufactured by Hitachi High-Technologies Corporation).
The average particle diameter of the product was evaluated by using dynamic light scattering (Zetasizer Nano S manufactured by Malvern).

The concentration of a suspension having the product dispersed therein was evaluated with a gravimetric analyzer (Thermo plus TG8120 manufactured by Rigaku Corporation).

The measurement of the R was performed with the apparatus 1 and the apparatus 2. The apparatus 1 is an apparatus having such a configuration as described below.

An LED light source for excitation light having a wavelength of 340 nm was prepared, and a polarizing filter (manufactured by Sigmakoki Co., Ltd., NSPFU-30C) and a shortpass filter (manufactured by Edmund Optics, Inc., 84-706) were inserted into an optical path to set an optical system capable of irradiating a 1 cm quartz square cell with the light. A polarizing filter (manufactured by Thorlabs, Inc., PIVISC050) and a bandpass filter (manufactured by Thorlabs, Inc., FB610-10) were set in a direction of 90° with respect to incident light. To measure luminescence as $I_{VV}$ and $I_{VH}$ in two directions at the same time, two sets in which the construction of a polarizer was changed by a direction of 90° with respect to incident light were prepared. For the detection of polarized light, spectrometry was performed with QEPRo manufactured by Ocean Optics, Inc. A temperature controller was set in a sample holder so that the polarization anisotropic property R was able to be measured at 37°C. The polarization anisotropic property R was measured while the output of the LED light source was fixed at 12 mW, and a cumulative time was set to 3 seconds. A measurement interval was set to 15 seconds. Based on the luminescence spectrum of the polarized luminescence thus obtained, the polarization anisotropic property R was determined by substituting a luminescence intensity in the wavelength range of from 600 nm or more to 630 nm or less into the equation (4).

The apparatus 2 used in the evaluations of the polarization anisotropic properties R and CRP antigen-antibody reactions of the products is an apparatus having such a configuration as described below.

An LED light source for excitation light having a wavelength of 340 nm was prepared, and a polarizing filter (manufactured by Sigmakoki Co., Ltd., NSPFU-30C) and a shortpass filter (manufactured by Edmund Optics, Inc., 84-706) were inserted into an optical path to set an optical system capable of irradiating a quartz cell having an optical path length of 5 mm with the light. Polarized luminescence generated from a sample was dispersed into two directions by setting, in the optical path on the same straight line as that of the excitation light across the sample, an excitation light-cutting filter (manufactured by Edmund Optics, Inc., 33-910), a polarized light beam splitter (manufactured by Edmund Optics, Inc., 47-127), and a polarizer (manufactured by Sigmakoki Co., Ltd., SPF-30C-32) in the stated order. The dispersed polarized luminescence (two directions) was detected with an avalanche photodiode (APD, manufactured by Hamamatsu Photonics K.K., C15522-3010SA). A temperature controller was set in a sample holder so that the polarization anisotropic property R was able to be measured at 37°C. The polarization anisotropic property R was measured while the output of the LED light source was fixed at 60 mW, and a cumulative time was set to 8 milliseconds. A measurement interval was set to 30 seconds. The signal of the polarized luminescence thus obtained was measured with an oscilloscope, and the polarization anisotropic property R was determined by substituting the signal into the equation (1).

The nonspecific agglutination suppression evaluation of the product was performed as described below.

60 µl of a human serum solution diluted 15-fold with a buffer solution was added to the luminescent reagent dispersion (3 mg/mL), and the mixture was kept at a temperature of 37°C for 5 minutes. An absorbance at 527 nm was measured before and after the temperature keeping, and the amount of change in absorbance before and after the temperature keeping was measured 3 times. Table 1 shows the average value of the 3 times. The evaluation was performed as follows: when the amount of change in "absorbance×10,000" value was less than 1,000, it was determined that nonspecific agglutination was suppressed, and when the amount was 1,000 or more, it was determined that nonspecific agglutination occurred.

(Performance Evaluation)

The synthesized luminescent reagent had a particle diameter of about 100 nm, and showed strong red luminescence with excitation light having a wavelength of 340 nm.

According to the results of the nonspecific agglutination suppression evaluation, the change in absorbance was equal to or less than the specific numerical value (the amount of change in "absorbance×10,000" value was 1,000 or less), and hence it was recognized that the particles were capable of suppressing nonspecific adsorption.

FIG. 5 shows the measurement results of Example 1. A period of time required to reach the R1 is plotted against an axis of abscissa, and the measured CRP concentration is plotted against an axis of ordinate. As can be seen from the figure, when the R1 is set to 0.062, the period of time required for the R value to reach 0.062 lengthens in accordance with the CRP concentration. It can be recognized from FIG. 5 that a region corresponding to a CRP antigen measurement range of from 1,000 pM to 5 pM can be measured in a measurement time of 400 seconds.

The results of Examples 1 to 4 are shown in Table 1. An initial polarization anisotropic property R0 measured only with the luminescent agent in each of Example 1 and Example 3 investigated with the apparatus 1 was 0.0585, and the polarization anisotropic property R0 investigated with the apparatus 2 was 0.0388. The difference reflects a difference in light-receiving sensitivity between the respective polarized light components of the apparatus, and does not affect the essence of the measurement. In Example 4, a polarization anisotropic property was investigated with the apparatus 1. As a result, an initial polarization anisotropic property R0 measured only with the luminescent agent was 0.0740. It is because sodium alginate having a thickening effect was added into the reagent that the R0 of Example 4 is larger than that of Example 1. Meanwhile, the value R2 of a saturated polarization anisotropic property was determined from a sample left to stand overnight with a large excess of the CRP antigen (100,000 pM). As a result, the initial polarization anisotropic property R2 measured only with the luminescent agent in each of Example 1, Example 3, Example 4, Comparative Example 1, and Comparative Example 2 investigated with the apparatus 1 was 0.1150, and the polarization anisotropic property R2 investigated with the apparatus 2 was 0.0900. It was recognized in each of Examples that the preset numerical value of the polarization anisotropic property R1 was present between the R0 and the R2.

In Example 1, the measurement was performed while the R1 was set to 0.062. As a result, it was recognized that the detection concentration of the CRP antigen ranged from 5 pM to 1,000 pM.

In Example 2, the measurement was performed while the R2 was set to 0.043. As a result, it was recognized that the detection concentration of the CRP antigen ranged from 0.16 pM to 200 pM.

The difference of this Example may be due to the sensitivity of a measuring instrument and a difference in preset condition for the value of the R1.

In Example 3, the antigen-antibody reaction was further evaluated separately for two measurement times. As can be seen from the results of Example 3, when the measurement time was a period of time as short as 5 minutes, measurement sensitivity in a low-concentration region (about 5 pM) was not obtained (from 50 pM to 1,000 pM), and in contrast, when the measurement time was as long as 40 minutes, the antigen-antibody reaction in a high-concentration region (about 1,000 pM) was saturated, and hence the polarization anisotropic property R did not increase so as to be equal to or more than the value of the R2 (from 5 pM to 200 pM). In other words, it was revealed that a measurement range narrowed in the result of a quantitative evaluation in one period of time like Comparative Examples, and it was revealed that when the R value was measured and quantitatively evaluated for a plurality of measurement times, the measurement was able to be performed in a wide measurement range (from 5 pM to 1,000 pM).

In Example 4, the CRP antigen concentration was evaluated under a state in which the concentration of the luminescent reagent to be used was reduced, and sodium alginate serving as a thickener was added. The value of the R2 was set to 0.0760. As a result, it was recognized that the detection concentration of the CRP antigen ranged from 0.05 pM to 100 pM in measurement at up to 600 seconds. It was revealed that when the concentration of the luminescent reagent was reduced, even a slight antigen-antibody reaction made the amount of change in polarization anisotropic property appear to be large, and that a promoting effect on the aggregation reaction of the particles in the luminescent reagent exhibited by the addition of the thickener enabled not only measurement in a wide range but also high-sensitivity and short-time measurement.

Table 1

| | Example 1 | Example 2 | Example 3 | | | Example 4 |
|---|---|---|---|---|---|---|
| Concentration of affinity particles | 0.01 mg/mL | 0.01 mg/mL | 0.01 mg/mL | 0.01 mg/mL | 0.01 mg/mL | 0.0025 mg/mL |
| Value of initial anisotropy $r_0$ | 0.0585 | 0.0388 | 0.0585 | 0.0585 | 0.0585 | 0.074 |
| Value of specific anisotropy $r_1$ | 0.062 | 0.043 | - | - | - | 0.076 |
| Value of saturated anisotropy $r_2$ | 0.115 | 0.09 | 0.115 | 0.115 | 0.115 | 0.115 |
| Period of time in which measurement is separately performed | - | - | 5 minutes -1 minute 40 minutes -1 minute | 5 minutes -1 minute | 40 minutes -1 minute | - |
| CRP antigen concentration range | 5 pM-1,000 pM | 0.16 pM-200 pM | 5 pM-1,000 pM | 50 pM-1,000 pM | 5 pM-200 pM | 0.05 pM-100 pM |

It was revealed from the foregoing that the analysis method according to this embodiment was a method by which the concentration of the CRP antigen serving as a target substance was able to be measured through use of one reagent composition with high sensitivity and in a wide measurement range.

Accordingly, the use of the analysis method according to this Example enables the measurement of the concentration of the target substance in a wide measurement range without preparation of many test reagents in accordance with the concentration of the target substance. In general, the preparation of many reagents for the same kind of target substance in an analysis apparatus often becomes a problem because the preparation is not only complicated but also affects a setting space or the like. In addition, when the concentration exceeds a measurement concentration range, and hence the quantification of the substance ends in failure, the dilution and preparation of a specimen need to be performed again, and hence cost for one measurement increases. Accordingly, the analysis method according to this Example that may be capable of covering a wide measurement range with a small number of kinds of reagents is useful as an approach for a specimen test.

The present invention is not limited to the embodiments described above, and various changes and modifications may be made without departing from the spirit and scope of the present invention. The following claims are thus appended hereto in order to make the scope of the present invention public.

The present application claims priority based on Japanese Patent Application No. 2022-079524 filed on May 13, 2022, and Japanese Patent Application No. 2023-076794 filed on May 8, 2023, and the entire contents thereof are incorporated herein by reference.

**Claims**

1. An analysis method including measuring a value (R) for polarization anisotropy through use of a luminescent reagent that binds to a target substance, to thereby calculate a concentration of the target substance, the analysis method comprising:

   a mixing step of mixing a sample containing the target substance and the luminescent reagent to provide a mixed liquid; and
   a measuring step of measuring the R of the mixed liquid after a lapse of a time T from a time of the mixing,
   wherein in the measuring step, a time at which the R reaches R1 is represented by T1, and the concentration of the target substance is calculated based on the T1, and
   wherein when the R measured for the luminescent reagent that is free from being mixed with the target substance is represented by R0, R1>R0 is satisfied.

2. The analysis method according to claim 1, wherein R1-R0≥0.0001 is satisfied.

3. The analysis method according to claim 1, wherein R0≥0.001 is satisfied.

4. The analysis method according to claim 1, wherein the luminescent reagent contains luminescent particles.

5. The analysis method according to claim 4, wherein the luminescent particle contains a europium complex.

6. The analysis method according to claim 1, wherein the luminescent reagent contains a ligand that binds to the target substance.

7. The analysis method according to claim 1, wherein the R is defined to be "r" in the following equation (1):
   [Math. 1]

$$r = \frac{I_{VV} - G * I_{VH}}{I_{VV} + 2 * G * I_{VH}}$$

$$G = \frac{I_{HV}}{I_{HH}} \qquad \cdots (1)$$

(in the equation (1),

   $I_{VV}$ represents a luminescence intensity of a luminescence component having a vibration direction parallel to that of a first polarized light beam at a time of excitation by the first polarized light beam,
   $I_{VH}$ represents a luminescence intensity of a luminescence component having a vibration direction orthogonal to that of the first polarized light beam at the time of excitation by the first polarized light beam,
   $I_{HV}$ represents a luminescence intensity of a luminescence component having a vibration direction orthogonal to that of a second polarized light beam having a vibration direction orthogonal to that of the first polarized light beam at a time of excitation by the second polarized light beam,

$I_{HH}$ represents a luminescence intensity of a luminescence component having a vibration direction parallel to that of the second polarized light beam having a vibration direction orthogonal to that of the first polarized light beam at the time of excitation by the second polarized light beam, and

G represents a correction value.)

8. The analysis method according to claim 1, wherein the measuring step is repeatedly performed.

9. The analysis method according to claim 8, wherein in the measuring step to be repeatedly performed, when the R exceeds the R1 for the first time in an n-th measuring step, any numerical value between an ending time of an (n-1)-th measuring step and a starting time of an (n+1)-th measuring step is adopted as the T1.

10. The analysis method according to claim 8, wherein the measuring step to be repeatedly performed is repeated at a period of (a+b) seconds where "a" represents a period of time required for the measurement and "b" represents a measurement interval.

11. The analysis method according to claim 8, wherein in the measuring step to be repeatedly performed, when the R1 exceeds R2, which is a value defined in advance, at a time at which the R reaches the R1 for the first time, at least one of the "a" or the "b" is set short.

12. An analysis apparatus configured to measure a value (R) for polarization anisotropy through use of a luminescent reagent that binds to a target substance, to thereby calculate a concentration of the target substance, the analysis apparatus comprising:

a mixing portion configured to mix a sample containing the target substance and the luminescent reagent to provide a mixed liquid;

a measuring portion configured to measure the R of the mixed liquid after a lapse of a time T from a time of the mixing; and

a controlling portion,

wherein when a time at which the R reaches R1 in the measuring portion is represented by T1, the controlling portion is configured to calculate the concentration of the target substance based on the T1,

provided that when the R measured for the luminescent reagent that is free from being mixed with the target substance is represented by R0, R1>R0 is satisfied.

# FIG. 1

MIXING STEP OF MIXING SAMPLE CONTAINING
TARGET SUBSTANCE AND LUMINESCENT
REAGENT TO PROVIDE MIXED LIQUID

MEASURING STEP OF MEASURING VALUE (R)
FOR POLARIZATION ANISOTROPY
(REPEATEDLY PERFORMED AT PERIOD OF (a+b) SECONDS)

$R \geqq R1$

NO

YES

T1

# FIG. 2

MIXING STEP OF MIXING SAMPLE CONTAINING TARGET
SUBSTANCE AND LUMINESCENT REAGENT TO
PROVIDE MIXED LIQUID

MEASURING STEP OF MEASURING VALUE (R) FOR
POLARIZATION ANISOTROPY
(REPEATEDLY PERFORMED AT PERIOD OF (a+b) SECONDS)

$R \geqq R1$      NO

YES

$R1 > R2$      YES → SHORTEN PERIOD

NO

T1

# FIG. 3

MIXING PORTION CONFIGURED TO MIX SAMPLE
CONTAINING TARGET SUBSTANCE AND LUMINESCENT
REAGENT TO PROVIDE MIXED LIQUID

MEASURING PORTION CONFIGURED TO MEASURE
VALUE (R) FOR POLARIZATION ANISOTROPY
(AT PERIOD OF (a+b) SECONDS)

CONTROLLING PORTION

FIG. 4

# FIG. 5

## INTERNATIONAL SEARCH REPORT

International application No.

**PCT/JP2023/017784**

### A. CLASSIFICATION OF SUBJECT MATTER

***G01N 21/64***(2006.01)i; ***G01N 21/21***(2006.01)i; ***G01N 33/543***(2006.01)i
FI: G01N21/64 A; G01N33/543 575; G01N33/543 525C; G01N21/21 Z; G01N21/64 F

According to International Patent Classification (IPC) or to both national classification and IPC

### B. FIELDS SEARCHED

Minimum documentation searched (classification system followed by classification symbols)

G01N21/62-G01N21/74; G01N33/00-G01N33/98

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| A | JP 2022-046118 A (NITTO DENKO CORP.) 23 March 2022 (2022-03-23)<br>entire text, all drawings | 1-12 |
| A | JP 2000-187003 A (SAGAMI CHEM. RES. CTR.) 04 July 2000 (2000-07-04)<br>entire text, all drawings | 1-12 |
| A | JP 2893772 B2 (MITSUBISHI CHEMICAL CORP.) 24 May 1999 (1999-05-24)<br>entire text, all drawings | 1-12 |
| A | JP 03-52575 B2 (ABBOTT LAB.) 12 August 1991 (1991-08-12)<br>entire text, all drawings | 1-12 |

☐ Further documents are listed in the continuation of Box C.     ☑ See patent family annex.

| | |
|---|---|
| \* Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| **07 June 2023** | **20 June 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**
Information on patent family members

International application No.

**PCT/JP2023/017784**

| Patent document cited in search report | | | Publication date (day/month/year) | Patent family member(s) | Publication date (day/month/year) |
|---|---|---|---|---|---|
| JP | 2022-046118 | A | 23 March 2022 | (Family: none) | |
| JP | 2000-187003 | A | 04 July 2000 | (Family: none) | |
| JP | 2893772 | B2 | 24 May 1999 | (Family: none) | |
| JP | 03-52575 | B2 | 12 August 1991 | US 4516856 A entire text, all drawings | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP H0352575 B
- JP 2893772 B
- JP 2022079524 A
- JP 2023076794 A